(19) Europäisches Patentamt
European Patent Office
Office européen des brevets

(11) Publication number: **0 142 309 B1**

# (12) EUROPEAN PATENT SPECIFICATION

(45) Date of publication of patent specification: **03.01.90**

(51) Int. Cl.$^5$: **C 07 J 71/00**

(21) Application number: **84307432.9**

(22) Date of filing: **29.10.84**

## (54) Method of preparing steroid compoundshaving a reduced particle size form.

(30) Priority: **29.10.83 GB 8328929**

(43) Date of publication of application:
**22.05.85 Bulletin 85/21**

(45) Publication of the grant of the patent:
**03.01.90 Bulletin 90/01**

(84) Designated Contracting States:
**AT BE CH DE FR IT LI LU NL SE**

(56) References cited:
**EP-A-0 108 606**
**GB-A-1 123 770**
**GB-A-2 010 278**

(73) Proprietor: **STERWIN AG.**
**Zeughausgasse 9**
**CH-6300 Zug (CH)**

(72) Inventor: **Riley, Derek Charles**
**31 Plessey Crescent**
**Whitley Bay Tyne and Wear NE25 8PR (GB)**
Inventor: **Johnston, David**
**39 Ilford Road High West Jesmond**
**Newcastle upon Tyne NE2 3NX (GB)**

(74) Representative: **Green, Alan James et al**
**SANDERSON & CO. European Patent Attorneys**
**34, East Stockwell Street**
**Colchester Essex CO1 1ST (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 142 309 B1

**Description**

The present invention relates to a method of preparing steroid compounds in a form having a particle size within a desired and specific range.

In our British Specification No. 2,130,588, there are described and claimed, in particular for use in preparing pharmaceutical compositions having improved absorption characteristics, certain 2α-cyano-4α,5α-epoxy-3-oxo-steroid compounds, the compounds being in particulate form and consisting of particles having a mean equivalent sphere volume diameter of less than about 20 μm, at least 95% of the particles having a particle size of less than about 50 μm.

Our earlier Specification is particularly concerned with the steroid compound having the common name "trilostane". However, the earlier invention also embraces the steroid compound known by the common name "epostane", which is a compound having the formula:

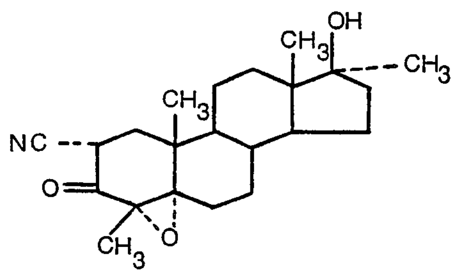

(I)

Furthermore, that compound known as "epostane" is also described in British Specification No. 2,010,278 where, in Example 1, step (f) and in Example 12 "epostane" is said to be recrystallised from hot dimethylformamide by addition of water.

Also, our earlier Specification No. 2,130,588 describes the preparation of, for example, trilostane in particulate form within the specified particle size range by the use of milling, preferably by the use of a fluid energy (air) mill under suitable conditions e.g. of air pressure and feed rate. In the case of epostane, however, we have observed that the raw compound is not easily reduced to a suitable particle size using a milling technique. Furthermore, in some instances milling can lead to a polymorphic change (which is undesirable), and/or to the formation of other impurities.

As a result of our study of the problem of preparing epostane in reduced particle size form, we have now found surprisingly that a more highly consistent material of reduced particle size can be produced, both in the case of epostane and in the case of like compounds, by first dissolving the compound in an organic solvent at a suitable concentration, and then precipitating the compound by mixing a non-solvent for the compound with the said solution, the mixing conditions being controlled to produce a precipitate having the desired reduced particle size.

Accordingly, the present invention provides a method of preparing a compound having a basic ring structure of the general formula:

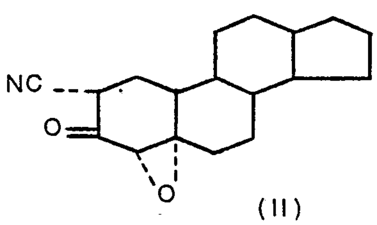

(II)

or

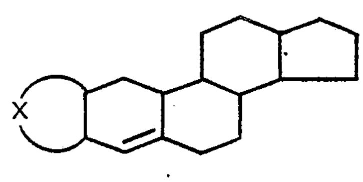

(III)

wherein X represents the remainder of a heterocyclic ring, in particular a 5-membered ring having at least two heteroatoms, or a compound of the formula:

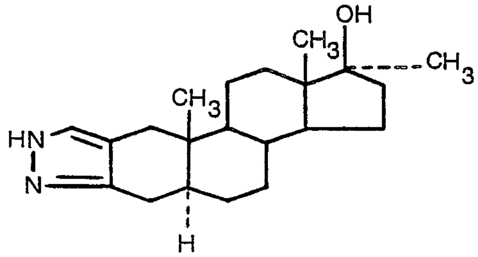

(VIII)

in a form having a reduced particle size characterised in that the compound is dissolved in an organic

solvent, the compound is precipitated from solution by mixing the thus-obtained solution and a non-solvent for the compound, and the mixing conditions are controlled so that the mixing period of time is 0.5 to 32 minutes and agitation is effected during mixing, thereby to produce a precipitate having a reduced particle size to provide a compound in a form consisting of particles having a mean equivalent sphere volume diameter of less than 20 µm, at least 95% of the particles having a particle size of less than 50 µm.

The method of the present invention may be used to prepare steroid compounds of the above formula II, III or VIII having a particle size reduced to varying degrees depending on the control exercised over the mixing conditions. Thus, the method may be used to prepare steroid compounds having a reduced particle size in accordance with British Specification No. 2,130,588, that is to say the method may be one in which the mixing conditions are controlled so that the chosen compound is prepared in a form comprising particles having a mean equivalent sphere volume diameter of less than 20 µm, at least 95% of the particles having a particle size of less than 50 µm. Furthermore, when following the teaching of the earlier invention, it is preferred that the mixing should be controlled so that the compound is prepared in a form consisting of particles having a mean equivalent sphere volume diameter within the various preferred limits disclosed in the earlier Specification, for example, preferably of from 4 to 12 µm, and more preferably of from 5 to 10 µm.

It is to be understood, however, that in the present invention the particle size of the compound may be outside the preferred ranges of the earlier invention. Thus, for example, as described specifically below particle sizes sometimes above 12 microns, e.g. 15 microns, but sometimes more generally below 5 microns may be obtained using the method of the invention.

Additionally or alternatively, and sometimes preferably, in the method of the present invention the mixing may be controlled so that the chosen compound is prepared in a form exhibiting a specific surface area of from 1 to 9 $m^2g^{-1}$. However, it is more preferred that the mixing is controlled so that the specific surface area of the chosen compound prepared by the method of the invention is from about 2 to about 4 $m^2g^{-1}$, especially in the case of epostane.

While a variety of organic solvents may be employed to dissolve compounds having a basic ring structure of the formula II, III or VIII set out above, the preferred organic solvents for use in the method of the present invention are acetone and dimethylformamide (DMF). In addition, it is preferred to use water as the non-solvent or a non-solvent comprising water, and the precipitating ability of the chosen non-solvent may be adjusted if desired or necessary by employing a suitable mixture of solvent e.g. DMF, and non-solvent e.g. water, as the precipitating medium.

In carrying out the method of the invention, the compound to be prepared in reduced particle size form is first dissolved in the chosen solvent. Generally, the compound will be dissolved in an amount with respect to the volume of solvent employed so as to give a dilution level which affords a reasonably wide variation in particle size of material thrown out of solution depending on the mixing conditions employed. Then, the desired particle size is obtained by controlling the mixing conditions, in particular the rate of mixing, to within the parameters which afford the desired particle size.

In order to achieve a suitable dilution, it may be necessary to choose with care the solvent in which the compound to be treated is to be dissolved. In that respect, we believe it is necessary for the compound to be at least reasonably soluble in the chosen solvent e.g. to a level of at least about 125 g/litre, and preferably at least about 300 g/litre, so as to provide at least some time lag between initial mixing of non-solvent and solution, and the commencement of precipitation. Thus, preferably the organic solvent and/or the dilution of the solution will be chosen so that the compound does not precipitate from the solution immediately on addition of the non-solvent, the delay in precipitation preferably being of the order of about 2 to about 3 minutes after mixing with the non-solvent is commenced.

The desired particle size characteristics are obtained by controlling the rate of mixing of the non-solvent with a solution in an organic solvent of the compound to be treated. In addition, it is preferred that the volume of non-solvent should be approximately equal to the volume of solvent, and mixing is preferably accomplished over a period of time of from about 4 to about 10 minutes. Thus, a typical preferred mixing time is about 6 minutes, and the necessary metering of non-solvent may conveniently be effected using a pump, for example, a peristaltic pump to supply the non-solvent to the solution. Alternatively, flow mixing (where the solution and solvent are blended in equal proportions) or inverse addition (where the solution is added to the non-solvent) may be employed.

In the method of the invention agitation is effected during mixing. It is preferred that at least the solution is agitated, preferably with high turbulence such as is obtained using a vibro-mixer. The degree of agitation necessary is to some extent governed by the rate of mixing of the non-solvent with the solution, and in order to ensure that a homogeneous mixture is produced it is preferred to increase the degree of agitation as the rate of mixing increases. It is believed that the rate of mixing determines the particle size, while the degree of agitation determines the uniformity of the product, i.e. the higher the degree of agitation the more uniform the product. However, provided those criteria are kept in mind the degree of agitation may be varied widely, for example, as represented by stirring speeds of from about 50 to about 500 rpm. Preferably also, the thus-formed liquid/solid suspension is stirred out under less turbulent conditions, preferably for a period of time up to 24 hours.

While the method of the invention is applicable to a variety of steroid compounds within the above formula II or III, the method is particularly applicable to the compounds commonly known as "trilostane"

and "epostane", those compounds falling within the formula:

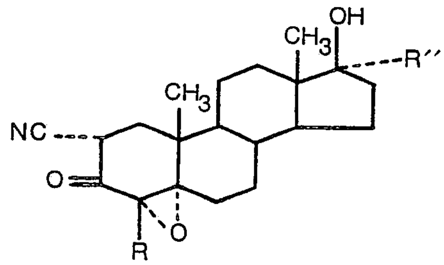

(IV)

wherein R is hydrogen or methyl and R" is hydrogen or lower alkyl.

In the above formula IV the compound known as "trilostane" is that wherein R and R" are hydrogen, and the compound known as "epostane" is that wherein R and R" are methyl.

Other specific compounds to which the method of the invention is applicable are:

1. The compound known as "nivacortol", that is a compound having the formula:

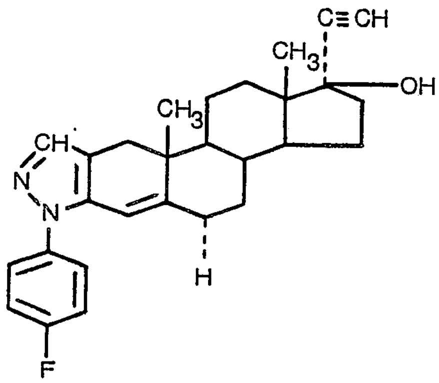

(V)

2. The compound known as "danazol", that is a compound having the formula:

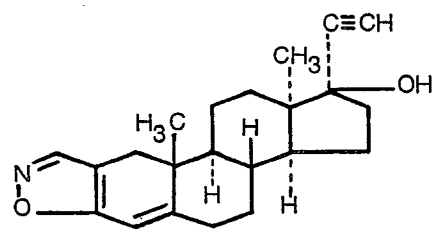

(VI)

3. The compound known as "17-ketotrilostane", that is a compound having the formula:

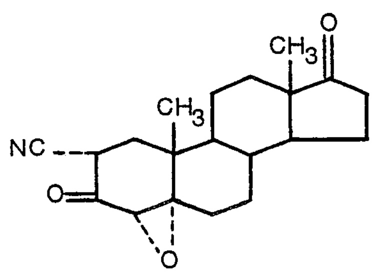

(VII)

as described in our co-pending British Patent Application No. 84—04980.

4. The compound known as "stanozolol", that is a compound having the formula:

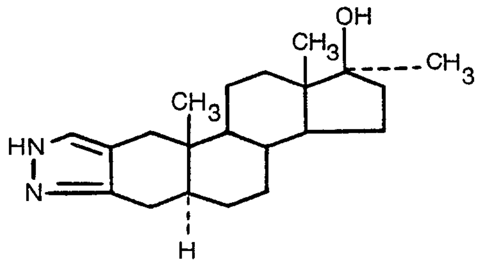

(VIII)

The method of the invention may include the step of isolating the compound prepared in the desired particle size form. Furthermore, the method may include one or more additional steps of blending the compound of reduced particle size with one or more pharmaceutically-acceptable excipients or carriers, and optionally thereafter bringing the thus-formed composition into a unit dosage form, for example, as a tablet, a capsule, a granulate for suspension, a cream, an ointment, a suppository or a suspension. In particular, the method of the invention may include the additional steps of granulation and terminal blending subsequent to isolation of the compound of reduced particle size.

The compound prepared in the desired particle size form may be isolated from the solid compound/solvent/non-solvent mixture by, for example, filtration, then washed and dried. A variety of drying techniques may be employed which may include one or more of suction drying, tray drying and oven drying. In addition, as may be necessary, one or more of the various steps of the method of the invention may be carried out under an inert atmosphere e.g. under an atmosphere of nitrogen.

In order to demonstrate the advantages of controlling the particle size of epostane and like steroid compounds a bioavailability study was carried out as follows:

The bioavailability of epostane from tablets containing a preferred raw material having a surface area in the range of from 2 to 4 $m^2g^{-1}$ i.e. 3.81 $m^2g^{-1}$, was compared in 8 male volunteers with that from tablets containing either a coarse grade material (surface area 0.56 $m^2g^{-1}$) or an especially fine grade material (surface area 7.01 $m^2g^{-1}$).

In the study, eight volunteers were dosed with epostane tablets (total dose 400 mg) on a cross-over basis, with 7 days between doses. Blood samples were taken before dosing and at 0.5, 1.0, 1.5, 2.0, 3.0, 4.0, 6.0, 8.0, 10.0, and 24.0 hours after dosing. Plasma was separated by centrifugation and stored at −15°C or lower until assay.

Plasma samples were analysed for epostane by HPLC (high pressure liquid chromatography) using a Partsil 5 C-8 RAC column. The eluent was 0.1M sodium acetate (pH 6.5):methanol (40:60 v/v) delivered at a flow rate of 2.0 $ml.min^{-1}$ and detection was at 280 nm.

The plasma epostane concentration data was processed using the programme 'BIOMU'. From an input of plasma concentrations by volunteer and part classification, and an input of the cross-over design, this programme produces tables of data according to formulation, determines the parameters $C_{max}$ and $t_{max}$ by inspection, calculates $AUC_0^t$ (where t is the last sampling point) using the trapezoidal rule, and performs statistical comparisons using the Student paired t-test.

From the results obtained, the tablets made from coarse grade material clearly gave lower plasma concentrations of epostane than the tablets made from preferred material as well as significant differences in the $C_{max}$ (P 0.01) and AUC (P 0.02) parameters, the mean results for each being:

| | $C_{max}$ | AUC |
|---|---|---|
| Coarse grade | 378.4 | 2115 |
| Preferred grade | 775 | 4147 |

Furthermore, the bioavailability from the coarse grade material was 51% of that of the preferred material.

In addition, while the mean plasma concentration profile for the fine grade material indicated quicker initial absorption from that material, in comparison with absorption from the preferred material, the only significant difference observed was in concentration at the 2.0 hour time point. Also, the mean values for $C_{max}$ and AUC were 613 and 3031 respectively for the fine grade material, and again the bioavailability from the tablets made from smaller particle size material was less, being only 74% that of the preferred material.

From the above results it will be seen that by using the method of the invention to produce say epostane having a particle size within the preferred range, a significant advantage can result in use of that compound in terms of the resulting bioavailability.

The method of the invention and compounds prepared thereby are illustrated in the procedures of the following specific Examples.

Example 1

3.49 kg of epostane were dissolved in 15 litres of Analar DMF at 25°C under an atmosphere of nitrogen gas to give an almost colourless solution. The solution was passed rapidly through a glass wool plug, in order to remove any undissolved particulate matter, into a 60 litre stainless steel container under a blanket

of nitrogen gas. The clear solution was rapidly stirred using a glass Herschberg stirrer. 15 litres of distilled water were added over a period of 30 seconds and the temperature of the thus-formed liquid/solid suspension rose to 37°C. The suspension was stirred out overnight under a blanket of nitrogen gas and the mixture was cooled to 0°C using a stainless steel flexiprobe cooler. The product was then recovered by filtration through a polypropylene filter cloth (smooth side facing upwards) on a bench top Buchner funnel and pressed dry. The product was washed with water (2 × 12.5 litres) at 17°C and dried under suction for 36 hours. The partially dry material was transferred to large stainless steel trays and dried in air for 5 days. The material was spread out thinly on the trays and the larger lumps of material were broken up at regular intervals (every 6 hours) to assist the drying process. After this time the material was dried *in vacuo* at 65°C for 24 hours, the temperature of the vacuum-oven being controlled using a 'fail-safe' Fi-monitor to ensure that the temperature of the oven did not exceed 65°C, since overheating of the oven is detrimental to the quality of the product.

Yield: 3.13 kg, 89.7%, m.p. 170.4 to 172°C.

The infra-red spectrum, differential scanning calorimeter trace and X-ray powder diffractogram of the thus-prepared material confirmed that the material had the desired polymorphic form (Form I).

Microscopic examination revealed a fairly narrow range of particle sizes up to approximately 15 µm with the vast majority of particles being less than 5 µm. Surface area measurements (Strohlein) gave duplicate values of 8.44 $m^2g^{-1}$ and 7.57 $m^2g^{-1}$.

Example 2

A 5.082 kg sample of epostane was divided into two batches each of 2.541 kg and each batch was dissolved in 10.92 litres of DMF under a flow of nitrogen gas at 60°C to give a dark brown, almost black coloured solution. When all of the epostane had dissolved, the two batches were combined in a large 60 litres stainless steel container under a blanket of nitrogen gas. The solution was stirred rapidly by a glass Herschberg stirrer and two Buchi "vibro-mixers" at full power. 21.73 litres of water were added over a period of 30 seconds and the final temperature of the thus-formed liquid/solid suspension was 46°C. The "vibro-mixers" were switched off after a uniform suspension was achieved (approximately 30 seconds). The thick, white suspension was stirred out overnight under a blanket of nitrogen gas and the mixture was simultaneously cooled to 0°C using a stainless steel flexiprobe cooler. The product was recovered by filtration of the suspension through a polypropylene filter cloth (smooth side facing upwards) on a benchtop Buchner funnel and washed with distilled water (60 litres) and pressed dry. The product was dried under suction for 24 hours and transferred to stainless steel trays and air-dried at ambient temperature for 5 days. The material was spread out thinly on the trays and the larger lumps of material were broken up at regular intervals (every 6 hours) to assist the drying process. The material was then transferred to a vacuum oven and dried in vacuo at 50 to 60°C for 36 hours to give a white, very fine crystalline powder of epostane, the temperature of the vacuum-oven again being controlled using a 'fail-safe' Fi-monitor to ensure that the temperature of the vacuum-oven did not exceed 65°C.

Yield: 4.91 kg, 97%, m.p. 169.8 to 171.4°C.

Infra-red spectrum and X-ray powder diffractogram data confirmed that the thus-formed epostane is of the desired polymorphic Form I.

Microscopic examination revealed a narrow distribution of particle sizes with the vast majority of particles less than 5 µm in size. Surface area measurements (Strohlein) gave duplicate values of 7.27 $m^2g^{-1}$ and 7.36 $m^2g^{-1}$.

Example 3

Effect of Addition Rate on Particle Size (Epostane)

For each of four experiments, epostane (250 g) was dissolved in DMF (1.05 litres) at 40°C in a 2 litre flat-topped reactor under a nitrogen atmosphere and the solution stirred at about 300 r.p.m. by a glass Herschberg stirrer driven by a Citenco overhead mechanical stirrer motor. The solution was allowed to cool within the range of from 30°C to 35°C and then water at room temperature (1.05 litres) was added over one of the following approximate periods, namely:

0.5 minutes — manually
2 minutes — via peristaltic pump
4 minutes — via peristaltic pump
32 minutes — via peristaltic pump

The suspensions resulting from the addition of water were stirred for a further hour under nitrogen and then the precipitated material was recovered by filtration. The residues, after washing with water (3 litres), were dried under reduced pressure overnight and then in a vacuum oven (approximately 2.6 mbar [2 mm Hg]) at 60°C for a further 24 hours.

All batches of material produced were shown to be predominantly of the desired end polymorph by means of infra-red spectroscopy and X-ray powder diffraction and all were acceptable analytically. The results of duplicate determinations of the surface areas (Strohlein) of the materials produced are given below:

| Addition time (minutes) | Surface Area ($m^2g^{-1}$) |
|---|---|
| 0.5 | 9.49, 9.80 |
| 2 | 5.34, 5.38 |
| 4 | 3.79, 3.83 |
| 32 | 1.72, 1.48 |

Example 4

500 g of epostane were dissolved in DMF (2.1 litres) at 40°C and the solution stirred under nitrogen as described in Example 3 above. Water (2.1 litres) at 25.5°C was added over 6 minutes *via* a previously calibrated Watson-Marlow 501 Hiflow peristaltic pump.

After the addition of water was complete, the suspension was stirred for a further hour. The epostane recovered by filtration through a polypropylene cloth was washed carefully with 6 litres of water. The filter cake was dried under suction overnight and then transferred to a vacuum oven where it was dried *in vacuo* (2.6 mbar [2 mm Hg]) for 4 hours at ambient temperature and 16 hours at 60°C.

The yield of the white crystalline product was 479 g (96%).

Duplicate values of 3.11 $m^2g^{-1}$ and 3.17 $m^2g^{-1}$ (Strohlein) were obtained on the material produced.

Example 5

2.1 kg of epostane were placed in a 20 litre flat top reactor, fitted with thermometer, nitrogen bleed and a Citenco overhead mechanical stirrer. DMF (8.82 litres) pre-heated to 40°C was added to the reactor under a blanket of nitrogen gas. The mixture was stirred at 300 r.p.m. until all the epostane had dissolved (3 to 4 minutes). The temperature of the DMF solution was 34.5°C. Water at 27.5°C was added at a rate of 1.47 litres $min^{-1}$ for 6 minutes.

Crystallisation commenced after 2.25 minutes. After the water addition was complete, the liquid/solid suspension was stirred out for 1 hour under a blanket of nitrogen gas.

The epostane was recovered by filtration on a polypropylene filter cloth (smooth surface upwards) and washed well with distilled water (1 × 20 litres, 1 × 12 litres, 2 × 4 litres). The material was dried under suction overnight and then transferred to stainless steel trays and air-dried for 3 days and finally dried under vacuum (2.6 mbar [2 mm Hg]) at 60°C for 16 hours.

To further batches of 2.1 kg and 1 kg were also processed as described above.

The three products thus-obtained were all of the desired polymorphic form, all had surface areas (Strohlein) in the range from about 3.1 to about 3.3 $m^2g^{-1}$ and recoveries ranged from 92 to 95.4%.

Example 6

Effect of Addition Rate on Particle Size (Trilostane)

For each of five experiments, trilostane (200 g) was dissolved in DMF (1.27 litres) at 60°C in a 2 litre flat-topped reactor under a nitrogen atmosphere and the solution stirred at about 200 r.p.m. by a glass Herschberg stirrer driven by a Buddeberg overhead air driven stirrer motor. The solution was allowed to cool within the range of from 30°C to 35°C and then water at room temperature (0.635 litres) was added over one of the following approximate periods using a previously calibrated peristaltic pump:

0.5 minutes
2 minutes
4 minutes
8 minutes
16 minutes

The suspensions resulting from the addition of water were stirred for a further hour under nitrogen and then the precipitated material was recovered by filtration. The residues after washing with 50% aqueous DMF (1 litre) and water (3 × 2 litres) were dried in a vacuum oven (approximately 2.6 mbar [2 mm Hg]) at 60°C for 16 hours.

All batches of material were shown to be acceptable analytically. The results of determinations of the surface areas (Strohlein) of the materials produced and the percentage recoveries are given below:

| Addition time (minutes) | Surface area ($m^2g^{-1}$) | (Recovery %) |
|---|---|---|
| 0.5 | 5.25 | 95.4 |
| 2 | 2.86 | 95.6 |
| 4 | 1.54 | 94.4 |
| 8 | 0.96 | 92.2 |
| 16 | 1.29 | 90.3 |

Example 7

Effect of Addition Rate on Particle Size of 17-ketotrilostane

For each of four experiments 17-ketotrilostane (20 g) was dissolved in DMF (140 ml) at 50°C in a 1 litre flat-topped reactor under a nitrogen atmosphere and the solution stirred at about 200 r.p.m. by a glass Herschberg stirrer driven by a Buddeberg overhead air driven stirrer motor. The solution was allowed to cool within the range of from 30°C to 35°C and then water at room temperature (140 ml) was added over one of the following approximate periods using a previously calibrated peristaltic pump:

0.5 minutes
2 minutes
8 minutes
16 minutes

The suspensions resulting from the addition of water were stirred for a further hour under nitrogen and then the precipitated material was collected by filtration. The residues after washing with 50% aqueous DMF (300 ml) and water (2 × 700 ml) were dried in a vacuum oven (approximately 2.6 mbar [2 mm Hg]) at 60°C for 16 hours. All batches of material produced were shown to be acceptable analytically. The results of the determinations of the surface areas (Strohlein) and the percentage recoveries of the materials produced are given below:

| Addition time (minutes) | Surface area ($m^2g^{-1}$) | (Recovery %) |
|---|---|---|
| 0.5 | 5.48 | 96 |
| 2 | 4.86 | 96 |
| 8 | 3.34 | 94 |
| 16 | 4.28 | 92.5 |

**Claims**

1. A method of preparing a compound having a basic ring structure of the general formula:

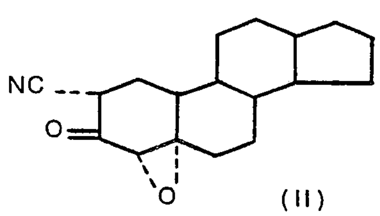

(II)

or

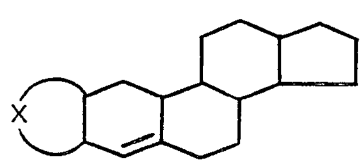

(III)

wherein X represents the remainder of a heterocyclic ring, in particular a 5-membered ring having at least 2 heteroatoms, or a compound of the formula:

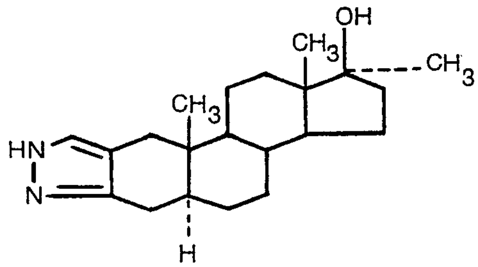

(VIII)

in a form having a reduced particle size characterised in that the compound is dissolved in an organic solvent, the compound is precipitated from solution by mixing the thus-obtained solution and a non-solvent for the compound, and the mixing conditions are controlled so that the mixing period of time is 0.5 to 32 minutes and agitation is effected during mixing, thereby to produce a precipitate having a reduced particle size to provide a compound in a form consisting of particles having a mean equivalent sphere volume diameter of less than 20 μm, at least 95% of the particles having a particle size of less than 50 μm.

2. A method according to claim 1, wherein the mixing conditions are controlled so that the compound is prepared in a form consisting of particles having a mean equivalent sphere volume diameter of from 4 to 12 μm.

3. A method according to claim 1 or claim 2, wherein the mixing is controlled so that the compound is prepared in a form exhibiting a specific surface area of from 1 to 9 $m^2g^{-1}$, and preferably a specific surface area of from 2 to 4 $m^2g^{-1}$.

4. A method according to any one of the preceding claims, wherein the organic solvent is dimethylformamide or acetone and/or the non-solvent is water.

5. A method according to any one of the preceding claims, wherein the rate of mixing of the non-solvent with the said solution is controlled to produce the desired particle size characteristics.

6. A method according to any one of the preceding claims, wherein a volume of non-solvent approximately equal to the volume of solvent is mixed with the solution over a period of time of from 4 to 10 minutes, and preferably of about 6 minutes.

7. A method according to any one of the preceding claims, wherein the solution is agitated during mixing and/or the liquid/solid suspension resulting from mixing is stirred out.

8. A method according to any one of the preceding claims, wherein the compound is epostane, trilostane, nivacortol, danazol, 17-ketotrilostane or stanozolol.

9. A method according to any one of the preceding claims, which includes the step of isolating the compound prepared in the desired particle size form, and the isolation preferably includes one or more drying steps selected from suction drying, tray drying and oven drying, the method preferably also including one or more additional steps of blending isolated compound of reduced particle size with one or more pharmaceutically-acceptable excipients or carriers, the blended compound preferably being brought into a unit dosage form such as a tablet, a capsule, a granulate for suspension, a cream, an ointment, a suppository or a suspension, and said additional steps more preferably including granulation and terminal blending subsequent to isolation of the compound of reduced particle size.

10. A method according to any one of the preceding claims, wherein the organic solvent and/or the dilution of the solution are chosen so that the compound does not precipitate from the solution immediately on mixing with the non-solvent, the delay in precipitation preferably being of the order of 2 to 3 minutes after mixing is commenced.

**Patentansprüche**

1. Eine Methode zur Herstellung einer Verbindung mit der grundlegenden Ringstruktur nach der allgemeinen Formel:

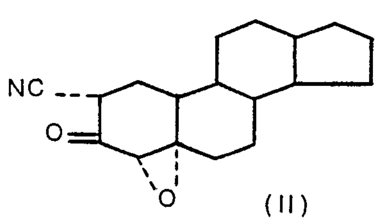

(II)

oder

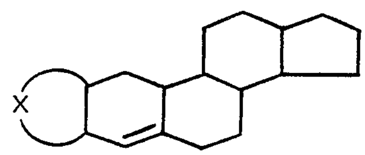

(III)

dadurch gekennzeichnet, dass x den Rest eines heterocyclischen Rings darstellt, insbesondere eines fünfgliedrigen Rings mit wenigstens zwei Heteroatomen oder eine Verbindung nach der Formel:

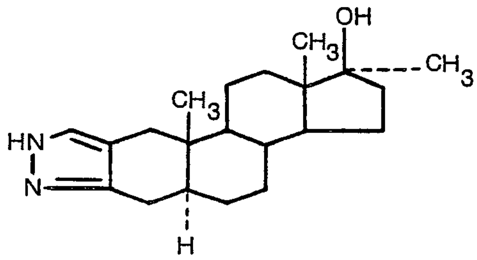

(VIII)

in einer Form, welche eine reduzierte Teilchengrösse aufweist, die dadurch gekennzeichnet ist, dass die Verbindung in einem organischen Lösungsmittel gelöst wird, die Verbindung von der Lösung getrennt wird, indem die so hergestellte Lösung und einen Nichtlöser für die Verbindung vermischt wird, und die Mischbedingungen so gesteuert werden, dass die Mischperiode zwischen 0,5 und 32 Minuten beträgt und der Schüttelvorgang während des Mischens erfolgt, dadurch gekennzeichnet, dass ein Niederschlag hergestellt wird, der eine reduzierte Teilchengrösse aufweist, die eine Verbindung in der Form von Teilchen mit einem durchschnittlichen volumetrischen Durchmesser von weniger als 20 µm gewährleistet, dadurch gekennzeichnet, dass wenigstens 95% der Teilchen eine Teilchengrösse von weniger als 50 µm aufweisen.

2. Eine Methode nach Anspruch 1, dadurch gekennzeichnet, dass die Mischbedingungen gesteuert werden, so dass die Verbindung eine Form aufweist, die aus Teilchen mit einem durchschnittlichen volumetrischen Durchmesser zwischen 4 und 12 µm besteht.

3. Eine Methode nach Anspruch 1 oder 2, dadurch gekennzeichnet, dass der Mischvorgang gesteuert wird, so dass die Verbindung in einer Form hergestellt wird, die eine spezifische Oberfläche zwischen 1 und 9 $m^2g^{-1}$ und vorzugsweise eine spezifische Oberfläche zwischen 2 und 4 $m^2g^{-1}$ aufweist.

4. Eine Methode nach jeglichem voranstehenden Anspruch, dadurch gekennzeichnet, dass das organische Lösungsmittel aus Dimethylformamyd oder Aceton besteht und/oder der Nichtlöser Wasser ist.

5. Eine Methode nach jeglichem voranstehenden Anspruch, dadurch gekennzeichnet, dass die Mischgeschwindigkeit des Nichtlösers mit besagter Lösung gesteuert wird, so dass die gewünschte Teilchengrösse hergestellt werden kann.

6. Eine Methode nach jeglichem voranstehenden Anspruch, dadurch gekennzeichnet, dass ein Volumen des unlöslichen Bestandteiles, welches ungefähr dem Volumen des Lösungsmittels entspricht, mit der Lösung während eines Zeitraumes zwischen 4 und 10 Minuten, vorzugsweise während eines Zeitraumes von 6 Minuten, gemischt wird.

7. Eine Methode nach jeglichem voranstehenden Anspruch, dadurch gekennzeichnet, dass die Lösung während des Mischvorganges geschüttelt wird und/oder die flüssige/feste Aufschwemmung, welche aus dem Mischvorgang hervorgeht, ausgeschüttelt wird.

8. Eine Methode nach jeglichem voranstehenden Anspruch, dadurch gekennzeichnet, dass die Verbindung aus Epostan, Trilostan, Nivacortol, Danazol, 17-Ketotrilostan oder Stanozolol besteht.

9. Eine Methode nach jeglichem voranstehenden Anspruch, dadurch gekennzeichnet, dass besagte Methode einen Verfahrensschritt einschliesst, indem die herzustellende Verbindung in der gewünschten Teilchengrösse isoliert wird und der Isolationsvorgang vorzugsweise einen oder mehrere Trocknungsvorgang/Trockungsvorgänge einschliesst, der/die aus den Trocknungsverfahren Saugtrockner, Schalentrockner und Wärmeschrank ausgewählt wird/werden, weiter dadurch gekennzeichnet, dass die Methode ebenfalls einen oder mehrere zusätzliche(n) Schritt(e) des Mischens der isolierten Verbindung aus der reduzierten Teilchengrösse mit einem oder mehreren pharmazeutisch vertretbaren Arzneistoffträger(n) oder Vehikel(n) umfasst, dadurch gekennzeichnet, dass die gemischte Verbindung zu einer Dosiereinheit, wie eine Tablette, eine Kapsel, eine Granulat, das zur Suspension bestimmt ist, eine Creme, eine Salbe, ein Suppositorium oder eine Suspension, verarbeitet wird, und dadurch gekennzeichnet, dass besagte(r) zusätzliche(r) Schritt(e) vorzugsweise eine Granulierung und ein abschliessendes Mischen nach der Isolation der Verbindung aus der reduzierten Teilchengrösse einschliesst.

10. Eine Methode nach jeglichem voranstehenden Anspruch, dadurch gekennzeichnet, dass das organische Lösungsmittel und/oder die Verdünnung der Lösung so gewählt wird/werden, dass die Verbindung nicht unmittelbar beim Mischen mit dem Nichtlöser getrennt wird und dass die zeitliche Verzögerung beim Trennvorgang vorzugsweise die Grössenordnung von 2 bis 3 Minuten nach dem Mischvorgang aufweist.

**Revendications**

1. Une méthode de préparation d'un composé ayant une structure cyclique de base dont la formule générale est:

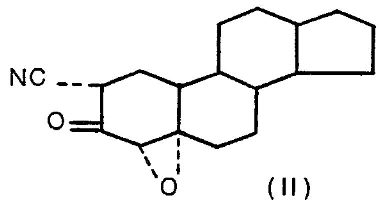

(II)

ou

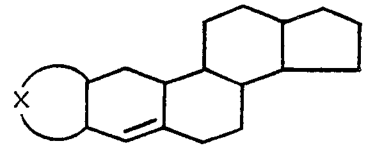

(III)

où X est le reste du noyau hétérocyclique, plus particulièrement un noyau à 5 chaînons ayant au moins 2 hétéroatomes, ou un composé dont la formule est:

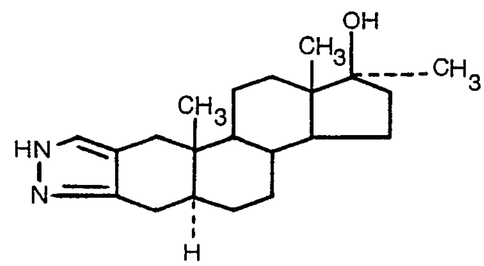

(VIII)

sous une forme ayant une granulométrie réduite, caractérisé par le fait que (a) le composé est solubilisé dans un solvant organique, (b) le composé est précipité de la solution par mélange de la solution qui vient d'être obtenue, avec un non-solvant pour le composé, et (c) les conditions de mélange sont contrôlées de manière à maintenir la durée du mélange entre 0,5 et 32 minutes, en effectuant en même temps une agitation du mélange, et par ceci de former un précipité ayant des dimensions des grains réduites pour obtenir (d) un composé sous forme des particules dont le diamètre du volume moyen de la sphère équivalente est inférieur à 20 µm, et au moins 95% des particules ont des dimensions inférieures à 50 µm.

2. Une méthode, selon la revendication 1, dans laquelle les conditions de mélange sont contrôlées de manière à préparer le composé sous la forme des particules dont le diamètre du volume moyen de la sphère équivalente est de 4 à 12 µm.

3. Une méthode selon la revendication 1 ou la revendication 2, dans laquelle le mélange est contrôlé de manière à préparer le composé ayant une valeur de la surface spécifique de 1 à 9 $m^2g^{-1}$, de préférence de 2 à 4 $m^2g^{-1}$.

4. Une méthode, selon l'une quelconque des revendications qui précèdent, dans laquelle le diméthylformamide ou l'acétone est le solvant organique, et/ou l'eau est le non-solvant.

5. Une méthode, selon l'une quelconque des revendications qui précèdent, dans laquelle la proportion du non-solvant dans le mélange avec la solution est contrôlée de manière à obtenir des dimensions de particules aux caractéristiques désirées.

6. Une méthode, selon l'une quelconque des revendications qui précèdent, dans laquelle le volume du non-solvant, à peu près égal au volume du solvant, est mélangé avec la solution pendant une période de 4 à 10 minutes, de préférence d'environ 6 minutes.

7. Une méthode, selon l'une quelconque des revendications qui précèdent, dans laquelle la solution est agitée pendant le mélangeage et/ou la suspension liquide/solide obtenue lors du mélangeage est remuée.

8. Une méthode, selon l'une quelconque des revendications qui précèdent, dans laquelle le composé est: l'épostane, le trilostane, le nivacortol, le danazol, le 17-kétotrilostane ou stanozolol.

9. Une méthode, selon l'une quelconque des revendications qui précèdent, qui (a) comporte le stade de la séparation du composé ayant les dimensions des particules désirées, ladite séparation comportant, de préférence, un ou plusieurs stades de séchage, sélectionné parmi le séchage à l'aspiration par le vide, le séchage aux étagères et le séchage au four, et (b) comporte également, de préférence, un ou plusieurs stades complémentaires de mélangeage du composé isolé ayant des dimensions des particules réduites, avec un ou plusieurs excipients ou supports acceptables pour l'application pharmaceutique, le composé mélangé étant, de préférence, mis en forme dans un dosage unitaire, comme comprimé, capsule, granulé pour suspension, crème, onguent, suppositoire ou suspension, et lesdits stades complémentaires comportant, de préférence, la granulation et le mélange final après l'isolation du composé à dimensions des particules réduites.

10. Une méthode, selon l'une quelconque des revendications qui précèdent, dans laquelle le solvant organique et/ou la dilution de la solution sont sélectionnés de manière à empêcher le composé de se précipiter de la solution immédiatement lors du mélange avec le non-solvant, le retardement de la précipitation étant de l'ordre de 2 à 3 minutes après le début du mélange.